(19) Europäisches Patentamt
European Patent Office
Office européen des brevets

(11) **EP 2 829 263 B1**

(12) **EUROPEAN PATENT SPECIFICATION**

(45) Date of publication and mention
of the grant of the patent:
**28.10.2020 Bulletin 2020/44**

(21) Application number: **13752537.4**

(22) Date of filing: **25.01.2013**

(51) Int Cl.:
*A61K 8/31* *(2006.01)*   *A61K 8/06* *(2006.01)*
*A61K 8/34* *(2006.01)*   *A61K 8/42* *(2006.01)*
*A61Q 19/00* *(2006.01)*

(86) International application number:
**PCT/JP2013/051672**

(87) International publication number:
**WO 2013/125291 (29.08.2013 Gazette 2013/35)**

(54) **OIL-IN-WATER EMULSION COMPOSITION**

ÖL-IN-WASSER-EMULSIONSVERBINDUNG

COMPOSITION D'ÉMULSION D'HUILE DANS L'EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.02.2012 JP 2012039370**

(43) Date of publication of application:
**28.01.2015 Bulletin 2015/05**

(73) Proprietor: **FUJIFILM Corporation
Tokyo 106-8620 (JP)**

(72) Inventor: **KITAOKA, Hiroyuki
Ashigarakami-gun
Kanagawa 258-8577 (JP)**

(74) Representative: **Hoffmann Eitle
Patent- und Rechtsanwälte PartmbB
Arabellastraße 30
81925 München (DE)**

(56) References cited:
**WO-A1-2009/080572      WO-A1-2010/134206
WO-A2-2006/069426      JP-A- H1 135 444
JP-A- H08 113 723      JP-A- 2000 229 827
JP-A- 2000 229 828      JP-A- 2003 212 709
JP-A- 2010 229 081**

Remarks:
The file contains technical information submitted after
the application was filed and not included in this
specification

**Description**

Technical Field

The present invention relates to an oil-in-water emulsion composition.

Background Art

In recent years, with a focus on the high functionalities of the carotenoids, various compositions containing carotenoid have been proposed. In general, carotenoid is widely known as a material sparingly soluble in water, and from the viewpoint of solubility and dispersibility of carotenoid, various techniques have been proposed.

For example, in Japanese Patent Application Laid-Open (JP-A) No. 2008-120712, there is a description of an aqueous dispersion for cosmetic products that contains carotenoid having substantially no vitamin A efficacy, and dihydric alcohol having 5 or more carbon atoms. In addition, in JP-A No. 2011-241177, there is a description of a method of obtaining a carotenoid-containing composition by heating an oil phase component mixture that includes a carotenoid component containing crystalline carotenoid, and specific (poly)glycerin fatty acid ester under a specific temperature condition.

SUMMARY OF INVENTION

Technical Problem

Meanwhile, among the carotenoids, it has become apparent that lycopene is a compound having poor stability and which is easily decomposed, and it has also become apparent that a stability of lycopene becomes particularly unstable depending on the combination with a specific compound which is widely used in various compositions from the viewpoint of antiseptic properties. Therefore, an oil-in-water emulsion composition that is excellent in storage stability while maintaining stability of lycopene is desired, but such a desired oil-in-water emulsion composition has not yet been provided under the current circumstances.

The invention has been made in a view of the above circumstances, and an object of the invention is to provide an emulsion composition in which decomposition of lycopene is suppressed, and which has excellent storage stability.

Solution to Problem

The means for solving the problems described above are as follows.

[1] An oil-in-water emulsion composition, including dispersed particles containing from 0.05% by mass to 2.5% by mass of lycopene; and at least one kind of compound selected from the group consisting of phenoxyethanol, an aliphatic diol compound having a carbon chain length of from 5 to 3 carbon atoms, an ether compound of glycerin and aliphatic alcohol, and a carbamic acid ester compound.

[2] The oil-in-water emulsion composition as described in [1], in which the dispersed particles have an average particle diameter of 100 nm or less.

[3] The oil-in-water emulsion composition as described in [1] or [2], in which the carbamic acid ester compound includes iodopropynyl butylcarbamate.

[4] The oil-in-water emulsion composition as described in any one of [1] to [3], in which the ether compound of glycerin and aliphatic alcohol includes ethylhexylglycerin.

[5] The oil-in-water emulsion composition as described in any one of [1] to [4], in which the aliphatic diol compound having a carbon chain length of from 5 to 3 carbon atoms includes at least one kind of aliphatic diol compound selected from octanediol or decanediol.

[6] An external preparation for skin, containing the oil-in-water emulsion composition described in any one of [1] to [5].

Advantageous Effects of Invention

According to the invention, an oil-in-water emulsion composition in which the decomposition of lycopene is suppressed, and the storage stability is excellent is provided.

DESCRIPTION OF EMBODIMENTS

[Oil-in-Water Emulsion Composition]

The oil-in-water emulsion composition of the invention (hereinafter sometimes referred to as "emulsion composition of the invention") is an oil-in-water emulsion composition including dispersed particles containing from 0.05% by mass to 2.5% by mass of lycopene; and at least one kind selected from the group consisting of phenoxyethanol, an aliphatic diol compound having a carbon chain length of from 3 to 10 carbon atoms, an ether compound of glycerin and aliphatic alcohol, and a carbamic acid ester compound (hereinafter sometimes referred to as "specific additive").

In the emulsion composition of the invention, the content of lycopene in the dispersed particles is set in the predetermined range, and at least one kind of the selected specific additive is contained, whereby decomposition of lycopene is effectively suppressed, and excellent storage stability is exhibited.

The emulsion composition of the invention is an oil-in-water emulsion composition obtained by the emulsification-mixing of an aqueous phase composition constituted by an aqueous phase component and an oil phase composition constituted by an oil phase component.

In the present invention, any numerical range expressed herein using "to" refers to a range including the numerical values before and after "to" as the minimum and maximum values, respectively.

In a case in which the amount of a component in the composition is indicated in the invention, when there are plural substances corresponding to the component in the composition, the indicated amount means the total amount of the plural substances present in the composition, unless specifically stated otherwise.

In the present invention, the term "aqueous phase" is used as a term contrasting with "oil phase", regardless of the type of solvent.

In the present specification, the term "process" encompasses an independent process, as well as a process that cannot be clearly distinguished from another process but yet achieves the expected effect of the process of interest.

In the present specification, the expression "(poly)glycerin fatty acid ester" includes all of the glycerin fatty acid esters containing one glycerin unit and one fatty acid unit, a glycerin fatty acid ester containing plural units of either one of the glycerin unit and the fatty acid unit, and a glycerin fatty acid ester containing plural units of both of the glycerin unit and the fatty acid unit. In the present specification, the expression "(poly)glycerin fatty acid ester" is used in the case of using these glycerin fatty acid esters without distinction.

In the invention, the term "dissolution temperature of lycopene" as used herein means the lowest temperature at which all of the crystals of lycopene are dissolved in a case in which a lycopene crystal or a composition containing a lycopene crystal is maintained at the temperature for 1 minute.

In addition, the term "storage stability" as used herein means that the stability of the emulsion composition is sustained without being impaired by the passage of time after the emulsion composition containing lycopene is prepared.

Each constituent element in the invention will be described in detail in the following.

<Dispersed Particles Containing Lycopene>

The emulsion composition of the invention includes dispersed particles containing from 0.05% by mass to 2.5% by mass of lycopene.

The dispersed particles in the invention are present as a dispersed phase (oil phase) in the emulsion composition of the invention that is an oil-in-water emulsion composition.

The content of lycopene in the dispersed particles is from 0.05% by mass to 2.5% by mass. From the viewpoint of exertion of the function expected of lycopene, the content is preferably 0.2% by mass or more, more preferably 0.5% by mass or more, and still more preferably 1.0% by mass or more. Further, from the viewpoint of suppression of the decomposition of lycopene, the content is preferably 2.2% by mass or less, more preferably 1.9% by mass or less, and still more preferably 1.6% by mass or less.

Further, the content of lycopene in the emulsion composition of the invention is preferably from 0.00001% by mass to 1% by mass, more preferably from 0.00005% by mass to 0.5% by mass, and still more preferably from 0.0001% by mass to 0.2% by mass with respect to the total mass of the emulsion composition. In a case in which the content of lycopene in the emulsion composition is in this range, the effect of lycopene can be more expected.

[Lycopene]

Lycopene is a carotenoid having a chemical formula of $C_{40}H_{56}$ (molecular weight of 536.87), belongs to a carotene that is one kind of carotenoid, and is a red pigment having an absorption maximum at 474 nm (acetone).

Lycopene is known to have an extremely high antioxidant effect, an extremely high whitening effect, and the like. The addition of lycopene to a food product, a cosmetic, a pharmaceutical raw material, a processed product thereof, and the like has been conventionally demanded, examined, and practiced.

Lycopene is also present in the form of a cis- or trans-isomer of conjugated double bond at the center of the molecule, and examples of the lycopene include an all-trans form, a 9-cis form, a 13-cis form, and the like. Any of these lycopenes may be used in the invention.

Lycopene may be used for the preparation of the emulsion composition of the invention as lycopene-containing oil or lycopene-containing paste, which is separated and extracted from a natural product containing lycopene.

In nature, lycopene is contained in a natural product such as a tomato, a persimmon, a watermelon, or a pink grapefruit. The lycopene-containing oil described above may also be the one separated and extracted from these natural products.

The form of the products containing lycopene is known to have 4 types of oil type, emulsion type, paste type, and powder type.

In addition, lycopene used in the invention may also be the one appropriately purified from an extract from a natural product, as necessary. Further, lycopene used in the invention may also be a synthetic product.

Examples of the one of the particularly preferred embodiments of lycopene in the invention include a fat-soluble extract extracted from tomato pulp. The fat-soluble extract extracted from the tomato pulp is particularly preferable from the viewpoint of stability, quality, and productivity of the composition containing the fat-soluble extract.

Herein, the fat-soluble extract extracted from tomato pulp means an extract extracted by using an oil solvent from the solid in a pulp form obtained by centrifugation of the pulverized material that is obtained by pulverization of tomatoes.

As the lycopene that is a fat-soluble extract, a tomato extract, which is widely commercially available as lycopene-containing oil or lycopene-containing paste, may be used. Examples of the commercially available tomato extract include, for example, Lyc-O-Mato 15% and Lyc-O-Mato 6%, available from Sunbright Co., Ltd., and LYCOPENE 18 available from KYOWA HAKKO BIO CO., LTD.

In the invention, another carotenoid component other than lycopene may be contained as long as the effect according to the invention is not impaired. Examples of the carotenoid component other than lycopene include specifically $\alpha$-carotene, $\beta$-carotene, $\gamma$-carotene, $\delta$-carotene, actinioerythrol, bixin, canthaxanthin, capsorubin, $\beta$-8'-apo-carotenal (apocarotenal), $\beta$-12'-apo-carotenal, a xanthophyll (for example, astaxanthin, fucoxanthin, lutein, zeaxanthin, capsanthin, $\beta$-cryptoxanthin, and violaxanthin), and a hydroxyl derivative or carboxyl derivative thereof. These carotenoids may be used alone, or in combination of two or more kinds thereof.

The lycopene in the invention is crystalline carotenoid.

Herein, the "crystalline carotenoid" does not refer to a specific carotenoid but means a carotenoid that can be present as a crystal at any temperature in a temperature range of from -5°C to 35°C, depending on various factors such as the production method, the treatment, and the storage, in the case of the form of oil, paste, or the like containing the carotenoid. In particular, lycopene that is contained in the emulsion composition of the invention, and $\beta$-carotene, $\delta$-carotene, zeaxanthin, lutein, astaxanthin, and the like that may be contained if desired, are carotenoids in which crystals are easily present.

The crystalline carotenoid is a pigment of a terpenoid with from yellow to red, and examples of the crystalline carotenoid may include those derived from a plant, an alga and a bacterium. In addition, the crystalline carotenoid is not limited to those derived from a natural product but may be any crystalline carotenoid as long as the crystalline carotenoid is obtained according to a usual method. Further, a crystalline carotenoid may be confirmed by a usual method, for which, for example, differential scanning calorimetric measurement (DSC: Differential scanning calorimetry), observation by a polarizing microscope, and X-ray diffraction can be used.

The carotenoid including lycopene in the invention is preferably contained in the emulsion composition of the invention as a crystalline carotenoid in a non-crystalline state. Since the crystalline carotenoid is in a non-crystalline state, the average particle diameter of dispersed particles can easily be 100 nm or less, and thus the absorbability of the carotenoid component into the body can be enhanced.

It can be confirmed using a known means for detecting a crystal structure that the crystalline carotenoid is non-crystalline. In addition, a crystalline carotenoid may be confirmed by a usual method, for which, for example, differential scanning calorimetric measurement (DSC: Differential scanning calorimetry), observation by a polarizing microscope, and X-ray diffraction can be used. According to the result that the detection of any crystals is not confirmed by these known techniques, the crystalline carotenoid can be demonstrated to be non-crystallinity. In particular, in the invention, it is preferable to confirm the non-crystallinity based on the presence of a DSC endothermic peak.

It is preferable to confirm that the crystalline carotenoid is in a non-crystalline state, specifically, by satisfying at least one of the conditions that using DSC Q2000 (TA Instruments Japan Inc.), in a state that the crystalline carotenoid has been freeze-dried to remove water, endothermic and exothermic temperatures are determined in one cycle of temperature-rise to temperature-fall (15°C/min) in the temperature range of from 30°C to 200°C, and the presence of a recognizable endothermic peak is not observed, and that when an emulsion in which the content of crystalline carotenoid is 0.1% by mass is observed by a polarizing microscope, the number of the crystals that can be confirmed in the field

4

of view of 1 cm $\times$ 1 cm is 1,000 or less.

In addition, in the carotenoid component contained in the invention, it is preferable that at least from 50% by mass to 100% by mass of the crystalline carotenoid is non-crystalline, it is more preferable that at least from 90% by mass to 100% by mass of the crystalline carotenoid is non-crystalline, and it is still more preferable that at least from 95% by mass to 100% by mass of the crystalline carotenoid is non-crystalline, in view of the dynamic absorbability.

It can be confirmed that the carotenoid component contains at least 50% by mass of the crystalline carotenoid in a non-crystalline state, for example, by the comparison of the endothermic quantity of the endothermic peak from a carotenoid crystal in the composition of the invention measured by differential scanning calorimetric measurement (DSC) with the endothermic quantity of the endothermic peak of a carotenoid crystal sample.

Further, it can also be confirmed by the comparison of the spectra of the emulsion composition of the invention with the spectra of a carotenoid crystal sample in X-ray diffraction.

In addition, the content ratio of a crystalline carotenoid that is non-crystalline, can be converted from a DSC peak area or the results obtained from XRD (X-ray diffraction) using a carotenoid reagent that is a crystal available as a commercial product, by setting the carotenoid reagent as 100%. Examples of the commercial product of a carotenoid reagent that is crystalline include, for example, a biochemical reagent available from Wako Pure Chemical Industries, Ltd.

The crystalline carotenoid may singly constitute the carotenoid component, or may constitute the carotenoid component together with the oil component (oil) used in the extraction from a natural product.

The carotenoid component may also contain a non-crystalline carotenoid (non-crystalline carotenoid) derived from a natural product, other than the crystalline carotenoids described above.

[Specific Additives]

The emulsion composition of the invention contains at least one kind of compound (specific additive) selected from the group consisting of phenoxyethanol, an aliphatic diol compound having a carbon chain length of from 3 to 10 carbon atoms, an ether compound of glycerin and aliphatic alcohol, and a carbamic acid ester compound. The specific additive is a compound contributing to the exhibition of antiseptic properties in the emulsion composition of the invention.

In the combination with the dispersed particles containing lycopene at prescribed amount, the specific additive can keep the favorable storage stability while suppressing the decomposition of lycopene in the emulsion composition.

In an aliphatic diol compound having a carbon chain length of from 3 to 10 carbon atoms, the carbon chain length means the number of the continuous carbon atoms that constitutes the aliphatic chain to which two hydroxy groups are bonded.

Examples of the aliphatic diol compound having a carbon chain length of from 3 to 10 carbon atoms include 1,3-butanediol, 1,2-pentanediol, 1,6-hexanediol, 1,2-heptanediol, 1,2-octanediol, 1,2-decanediol, and the like. From the viewpoint of particle diameter stability of the dispersed particles during the storage of the emulsion composition, 1,2-octanediol, 1,2-decanediol, and the like are more preferable.

Examples of the ether compound of glycerin and aliphatic alcohol include an ether compound of glycerin having from 6 to 11 carbon atoms in total and aliphatic alcohol. The ether compound may have a linear or branched chain, and for example, ethylhexylglycerin is preferable.

Examples of the carbamic acid ester compound include various compounds, and among them iodopropynyl butylcarbamate is preferable.

Among the specific additives, from the viewpoint of obtaining a superior effect by small amount usage, a carbamic acid ester compound is more preferable.

The emulsion composition of the invention may contain only one kind of specific additive, or may contain two or more kinds of specific additives in combination.

The content of specific additive can be from 0.001% by mass to 25% by mass, and more preferably from 0.003% by mass to 15% by mass relative to the total mass of the emulsion composition.

[(Poly)glycerin Fatty Acid Ester]

The emulsion composition of the invention preferably contains a (poly)glycerin fatty acid ester having an HLB of 6 or less as an oil phase component contained in the dispersed particles.

The (poly)glycerin fatty acid ester described above is preferably a (poly)glycerin fatty acid ester having from 1 to 3 glycerin units, from 1 to 6 fatty acid units, and at least one hydroxyl group from a glycerin unit.

In a co-dissolved material of such a specific (poly)glycerin fatty acid ester, and lycopene that is a crystalline carotenoid, recrystallization of the crystalline carotenoid is suppressed.

A (poly)glycerin fatty acid ester having 3 or less glycerin units has high affinity with a carotenoid such as lycopene. On the other hand, a (poly)glycerin fatty acid ester having 6 or less fatty acid units has a high crystal suppressing effect of carotenoid. In addition, the crystallization of carotenoid can be sufficiently suppressed by the inclusion of a (poly)glycerin fatty acid ester containing a hydroxyl group from a glycerin unit.

From the viewpoint of recrystallization suppression and the like, the (poly)glycerin fatty acid ester is preferably an ester of glycerin having from 1 to 3 (more preferably from 1 to 2) glycerin units (average degree of polymerization), and a fatty acid having from 1 to 6 (more preferably from 1 to 5) fatty acid units and having from 8 to 22 carbon atoms (more preferably a fatty acid having from 14 to 18 carbon atoms). Examples of the fatty acid having from 8 to 22 carbon atoms include, for example, caprylic acid, capric acid, lauric acid, myristic acid, palmitic acid, stearic acid, oleic acid, linoleic acid, and behenic acid.

Among these (poly)glycerin fatty acid esters, from the viewpoint of homogeneous solubility during co-dissolution, the molecular weight of the (poly)glycerin fatty acid ester is preferably 10,000 or less, more preferably 3,000 or less, and still more preferably 2,500 or less.

Further, from the viewpoint of affinity with lycopene, the (poly)glycerin fatty acid ester preferably has an HLB of 9 or less, and more preferably an HLB of 6 or less.

Examples of the (poly)glycerin fatty acid ester that can be used in the emulsion composition of the invention include, for example, glyceryl myristate, glyceryl monostearate, diglyceryl monostearate, triglyceryl monostearate, triglyceryl dipalmitate, glyceryl distearate, and hexaglyceryl tetrabehenate. From the viewpoint of recrystallization suppression and homogeneous solubility, glyceryl myristate, glyceryl monostearate, or diglyceryl monostearate is preferable.

From the viewpoint of stability of the emulsion composition, the content (mass) of the (poly)glycerin fatty acid ester is preferably from 0.01 times to 9 times, more preferably from 0.1 times to 8 times, and still more preferably from 0.3 times to 5 times the total mass of the lycopene.

In a case in which the total mass of the (poly)glycerin fatty acid ester in the emulsion composition is 0.01 times or more the total mass of the lycopene, a sufficient crystal suppressing effect can be expected. On the other hand, in a case in which the total mass of the (poly)glycerin fatty acid ester in the emulsion composition is 9 times or less the total mass of the lycopene, the increase in particle diameter of dispersed particles in the emulsion composition can be suppressed.

[Other Fatty Acid Esters]

The emulsion composition of the invention preferably contains at least one kind of fatty acid ester selected from the group consisting of a triester of glycerin and fatty acid, and an ester of alcohol having one hydroxyl group and fatty acid (hereinafter, sometimes referred to as "other fatty acid esters").

Other fatty acid esters are preferably contained as an oil phase component that is contained in the dispersed particles. The other fatty acid esters are at least one kind of fatty acid ester selected from the group consisting of a triester of glycerin and fatty acid, and an ester of alcohol having one hydroxyl group and fatty acid, and are preferably a fatty acid ester having from 10 to 60 carbon atoms in total that does not have a hydroxyl group in the molecule.

Such other fatty acid esters reduce the dissolution temperature of a crystalline carotenoid such as lycopene. Further, by containing other fatty acid esters, the fineness of the emulsified particles in the emulsion composition of the invention can be maintained stably.

In a case in which the total number of the carbon atoms in the other fatty acid esters is 10 or more, the increase in particle diameter of the dispersed particles that have been made into emulsion tends to be suppressed. Further, in a case in which the total number of the carbon atoms is 60 or less, the dissolution temperature of crystalline carotenoid tends to be sufficiently lowered.

From the viewpoint of decrease of the dissolution temperature of crystalline carotenoid, the total number of carbon atoms is preferably from 10 to 60, and more preferably from 27 to 57 in the other fatty acid esters.

Further, from the viewpoint of suppression of increasing a particle diameter of the dispersed particles that have been made into emulsion, each fatty acid unit in other fatty acid esters is preferably a fatty acid unit having from 8 to 18 carbon atoms, more preferably a fatty acid unit having from 8 to 12 carbon atoms, and still more preferably a fatty acid unit having from 8 to 10 carbon atoms.

From the viewpoint of decreasing the dissolution temperature of crystalline carotenoid, the total number of carbon atoms is preferably from 10 to 60, and more preferably from 27 to 57 in the triester of glycerin and fatty acid.

From the viewpoint of suppression of increasing a particle diameter of the dispersed particles that have been made into emulsion, each of three fatty acid units in the triester of glycerin and fatty acid is preferably a fatty acid unit having from 8 to 18 carbon atoms, more preferably a fatty acid unit having from 8 to 12 carbon atoms, and still more preferably a fatty acid unit having from 8 to 10 carbon atoms. The fatty acid unit may be a fatty acid unit derived from saturated fatty acid, or may be a fatty acid unit derived from unsaturated fatty acid. Further, the fatty acid unit may be a fatty acid unit derived from straight chain fatty acid, or may be a fatty acid unit derived from branched chain fatty acid. Among them, from the viewpoint of decreasing the dissolution temperature of crystalline carotenoid such as lycopene, the fatty acid unit is preferably a fatty acid unit derived from straight chain fatty acid.

Examples of the triester of glycerin and fatty acid include specifically, for example, glyceryl tricaprylate, glyceryl tricaprate, glyceryl trilaurate, glyceryl trimyristate, glyceryl tripalmitate, glyceryl tripalmitoleate, glyceryl tristearate, glyceryl trioleate, glyceryl trilinoleate, glyceryl trilinolenate, and tri(caprylic acid/capric acid)glyceryl.

From the viewpoint of decreasing the dissolution temperature of crystalline carotenoid, glyceryl tricaprylate, glyceryl tricaprate, glyceryl trilaurate, tri(caprylic acid/capric acid)glyceryl, and the like are preferable.

The triester of glycerin and fatty acid may be used singly, or may be used in combination of two or more kinds thereof.

In addition, olive oil, camellia oil, macadamia nut oil, castor oil, avocado oil, evening primrose oil, turtle oil, corn oil, mink oil, rapeseed oil, egg yolk oil, sesame oil, persic oil, wheat germ oil, sasanqua oil, linseed oil, cotton seed oil, perilla oil, soybean oil, tea seed oil, kaya oil, rice bran oil, china wood oil, Japan tung oil, jojoba oil, germ oil, glycerin trioctanoate, glycerin triisopalmitate, salad oil, safflower oil (Carthamus tinctorius oil), coconut oil, peanut oil, almond oil, hazelnut oil, walnut oil, grape seed oil, cacao butter, palm oil, shortening, and the like, which are a mixture of a triester of glycerin and fatty acid, can be used.

From the viewpoint of suppression of increasing a particle diameter of the dispersed particles that have been made into emulsion, the total number of carbon atoms is preferably from 10 to 50, and more preferably from 10 to 30 in the ester of alcohol having one hydroxyl group and fatty acid.

From the viewpoint of decrease of the dissolution temperature of crystalline carotenoid such as lycopene, the fatty acid unit in the ester of alcohol having one hydroxyl group and fatty acid is preferably a fatty acid unit having from 8 to 18 carbon atoms, more preferably a fatty acid unit having from 8 to 12 carbon atoms, and still more preferably a fatty acid unit having from 8 to 10 carbon atoms. The fatty acid unit may be a fatty acid unit derived from saturated fatty acid, or may be a fatty acid unit derived from unsaturated fatty acid. Further, the fatty acid unit may be a fatty acid unit derived from straight chain fatty acid, or may be a fatty acid unit derived from branched chain fatty acid. Among them, from the viewpoint of decreasing the dissolution temperature of crystalline carotenoid such as lycopene, the fatty acid unit is preferably a fatty acid unit derived from straight chain fatty acid.

From the viewpoint of decreasing the dissolution temperature of crystalline carotenoid, the alcohol unit in the ester of alcohol having one hydroxyl group and fatty acid is preferably an alcohol unit having from 2 to 35 carbon atoms, more preferably an alcohol unit having from 4 to 20 carbon atoms, and still more preferably an alcohol unit having from 5 to 15 carbon atoms. The alcohol unit may be an alcohol unit derived from saturated alcohol, or may be an alcohol unit derived from unsaturated alcohol. Further, the alcohol unit may be an alcohol unit derived from straight chain alcohol, or may be an alcohol unit derived from branched chain alcohol. Among them, from the viewpoint of decreasing the dissolution temperature of crystalline carotenoid, the alcohol unit is preferably an alcohol unit derived from straight chain alcohol.

Examples of the ester of alcohol having one hydroxyl group and fatty acid include, for example, hexyl caprylate, hexyl laurate, methylheptyl laurate, octyldodecyl myristate, methylheptyl isostearate, isocetyl isostearate, methylheptyl isostearate, isopropyl isostearate, butyl stearate, and 2-ethylhexyl stearate. From the viewpoint of decreasing the dissolution temperature of crystalline carotenoid, methylheptyl laurate, methylheptyl isostearate, and the like are preferable.

The ester of alcohol having one hydroxyl group and fatty acid may be used singly, or may be used in combination of two or more kinds thereof.

Further, the other fatty acid esters may be used in combination of two or more kinds, regardless of the kind of the triester of glycerin or fatty acid and the ester of alcohol having one hydroxyl group and fatty acid.

From the viewpoint of decreasing the dissolution temperature of lycopene in the emulsion composition, the content (mass) of the other fatty acid esters is preferably from 3 times to 300 times, more preferably from 5 times to 200 times, and still more preferably from 7 times to 100 times the total mass of the lycopene.

In a case in which the content of the other fatty acid esters in the emulsion composition of the invention is 3 or more times the total mass of the lycopene, a sufficient crystal suppressing effect can be expected. On the other hand, in a case in which the content of the other fatty acid esters is 300 or less times the total mass of the lycopene, the formulation of lycopene in a sufficient amount cannot be impaired.

The content (mass) of the other fatty acid esters varies even depending on the kind or content of the (poly)glycerin fatty acid ester to be used. From the viewpoint of stability of the emulsion composition, the content (mass) of the other fatty acid esters is preferably from 0.8 times to 750 times, more preferably from 1 time to 300 times, and still more preferably from 2 times to 100 times the total mass of the (poly)glycerin fatty acid ester.

In a case in which the content of the other fatty acid esters in the emulsion composition is 0.8 or more times the total mass of the (poly)glycerin fatty acid ester, a sufficient stability enhancement effect of the emulsion composition can be expected. On the other hand, in a case in which the content of the other fatty acid esters is 750 or less times the total mass of the (poly)glycerin fatty acid ester, the formulation of a carotenoid component such as lycopene in a sufficient amount cannot be impaired.

In the invention, it is preferable that the other fatty acid esters are a triester of glycerin and fatty acid, the content (mass) of the other fatty acid esters is from 7 times to 100 times the crystalline carotenoid, the total mass of the (poly)glycerin fatty acid ester is from 0.3 times to 5 times the total mass of the lycopene, and the content (mass) of the other fatty acid esters is from 2 times to 100 times the total mass of the (poly)glycerin fatty acid ester, in view of the crystallization suppression and stability of carotenoid component.

[Other Oil Components]

The dispersed particles in the emulsion composition of the invention may contain other oil components in addition to each of the components that have already been mentioned in the above.

As the other oil components, which are not particularly limited as long as the solubility into water at 25°C is less than 0.5%, and 5% or more of the oil component is dissolved at 90°C into the oil phase component containing lycopene in the invention, the oil component having physical properties and functionality depending on the purpose can be appropriately selected and used. As the other oil components, for example, an antioxidant, a non-crystalline carotenoid, an unsaturated fatty acid, squalane, squalene, a ubiquinone, and a fat-soluble vitamin are preferably used.

Examples of the unsaturated fatty acids include, for example, monovalent highly unsaturated fatty acid ($\omega$-9, oleic acid, and the like), and polyvalent highly unsaturated fatty acid ($\omega$-3, $\omega$-6), which have preferably 10 or more carbon atoms and more preferably from 18 to 30 carbon atoms. Such unsaturated fatty acids may be any of known unsaturated fatty acids. For example, examples of the $\omega$-3 oil or fats may include linolenic acid, eicosapentaenoic acid (EPA), docosahexaenoic acid (DHA), and fish oils containing these acids.

Examples of the ubiquinone include a coenzyme Q such as coenzyme Q10.

Further, examples of the fat-soluble vitamin may include a fatty acid ester of erythorbic acid such as erythorbyl palmitate, or erythorbyl tetraisopalmitate; and a fatty acid ester of vitamin B6 such as pyridoxine dipalmitate, pyridoxine tripalmitate, pyridoxine dilaurate, or pyridoxine dioctanoate.

[Antioxidant]

The emulsion composition of the invention preferably contains an antioxidant. The antioxidant may be a water-soluble antioxidant, or may be an oil-soluble antioxidant. The water-soluble antioxidant is preferably added as an aqueous phase component, and the oil-soluble antioxidant is preferably added as an oil phase component.

It is assumed that the decomposition (for example, oxidative decomposition) due to the heating of lycopene can be surely suppressed by containing an antioxidant, in the emulsion composition of the invention.

As the antioxidant, for example, among the various antioxidants described in "Kosankazai no Riron to Jissai (Theory and Practice of Antioxidants)" written by Kajimoto and published by San Shobo (1984), and Sanka Boshizai Handobukku (Handbook of Antioxidants) written by Sawatari, Nishino, and Tabata and published by Taiseisha (1976), any compound that functions as an antioxidant may be used. Specifically, the antioxidant is preferably at least one kind selected from the group consisting of a compound having a phenolic hydroxyl group, and an ascorbic acid compound. Hereinafter, examples of the preferred antioxidant will be described in the following, but are not limited thereto.

Examples of the compound having a phenolic hydroxyl group include an aromatic carboxylic acid, a cinnamic acid or an ellagic acid, BHT (butylhydroxytoluene), BHA (butylhydroxyanisol), and a vitamin E.

Examples of the aromatic carboxylic acid may include gallic acid (3,4,5-hydroxybenzoic acid), and a derivative thereof. Examples of the derivative of gallic acid (3,4,5-hydroxybenzoic acid) may include a gallic acid ester such as propyl gallate, epicatechin gallate, or epigallocatechin gallate; and a gallic acid glucoside such as gallotannin.

Examples of the cinnamic acid may include ferulic acid, chlorogenic acid, or the like, and a derivative thereof. Examples of the derivative of ferulic acid or chlorogenic acid may include a ferulic acid ester. The specific examples may include ferulic acid, $\gamma$-orizanol (rice bran extract), caffeic acid (coffeic acid, or 3,4-dihydroxycinnamic acid), chlorogenic acid, glyceryl ferulic acid, and dihydroferulic acid.

Examples of the ellagic acid may include ellagic acid.

Examples of the vitamin E include, but are not particularly limited to, a compound selected from the compound group consisting of tocopherol and a derivative thereof, or the compound group consisting of tocotrienol and a derivative thereof. These vitamins E may be used alone or in combination of plural kinds. Further, a compound selected from the compound group consisting of tocopherol and a derivative thereof, and a compound selected from the compound group consisting of tocotrienol and a derivative thereof may be used in combination.

Examples of the compound group consisting of tocopherol and a derivative thereof include dl-$\alpha$-tocopherol, dl-$\beta$-tocopherol, dl-$\gamma$-tocopherol, dl-$\delta$-tocopherol, dl-$\alpha$-tocopherol acetate, dl-$\alpha$-tocopherol nicotinate, dl-$\alpha$-tocopherol linoleate, and dl-$\alpha$-tocopherol succinate. Among them, dl-$\alpha$-tocopherol, dl-$\beta$-tocopherol, dl-$\gamma$-tocopherol, or dl-$\delta$-tocopherol, and a mixture thereof (mixed tocopherol) are more preferable. Further, as the tocopherol derivative, a carboxylic acid ester thereof, particularly an acetic acid ester thereof, is preferably used.

In the compound group consisting of tocotrienol and a derivative thereof, $\alpha$-tocotrienol, $\beta$-tocotrienol, $\gamma$-tocotrienol, and $\delta$-tocotrieno are included. Further, as the tocotrienol derivative, an acetic acid ester thereof is preferably used.

From the viewpoint of stability of the carotenoid including lycopene, the compound group consisting of tocopherol and a derivative thereof, or the cinnamic acid is preferable, as the compound having a phenolic hydroxyl group. Among them, mixed tocopherol, ferulic acid, or γ-orizanol, and a mixture thereof are more preferable.

As the molecular weight of the compound having a phenolic hydroxyl group, the low molecular weight is preferable from the viewpoint of stability of the carotenoid component including lycopene. For example, the compound having a phenolic hydroxyl group has preferably a molecular weight of from 100 to 3,000, and more preferably from 100 to 1,000.

The total amount of the phenolic hydroxyl group in the emulsion composition of the invention may be an effective amount for the suppression of the decomposition or disappearance of the carotenoid including lycopene. The total amount of the phenolic hydroxyl group can be from 1.3 times to 15.0 times, and is preferably from 2 times to 10 times and more preferably from 3 times to 8 times the amount of the carotenoid component including lycopene by molar ratio. In a case in which the total amount of the phenolic hydroxyl group is 1.3 or more times the amount of carotenoid component including lycopene by molar ratio, the effect of suppressing the decomposition or disappearance of the carotenoid component is sufficiently exhibited, and in a case in which the total amount of the phenolic hydroxyl group is 15.0 or less times, the formulation of a carotenoid component including lycopene in a sufficient amount cannot be impaired.

In the ascorbic acid compound, ascorbic acid, or an ascorbic acid ester, and a salt thereof are included.

Examples of the ascorbic acid compound include L-ascorbic acid, sodium L-ascorbate, potassium L-ascorbate, calcium L-ascorbate, L-ascorbyl phosphate, a magnesium salt of L-ascorbyl phosphate, L-ascorbyl sulfate, a disodium salt of L-ascorbyl sulfate, L-ascorbyl stearate, L-ascorbyl 2-glucoside, L-ascorbyl palmitate, and L-ascorbyl tetraisopalmitate; and also an ascorbic fatty acid ester such as L-ascorbyl stearate, L-ascorbyl tetraisopalmitate, or L-ascorbyl palmitate. Among them, from the viewpoint of heat loss suppression of the carotenoid, L-ascorbic acid, sodium L-ascorbate, calcium L-ascorbate, L-ascorbyl stearate, L-ascorbyl 2-glucoside, L-ascorbyl palmitate, a magnesium salt of L-ascorbyl phosphate, a disodium salt of L-ascorbyl sulfate, and L-ascorbyl tetraisopalmitate are particularly preferable.

These ascorbic acid compounds may be contained as a single substance in the oil phase component mixture, or may be mixed into the oil phase component mixture in the form of an aqueous solution. The concentration of such an ascorbic acid compound in an aqueous solution is, but not particularly limited to, generally preferably from 0.05% by mass to 5% by mass from the viewpoint of antioxidation properties.

From the viewpoint of suppression of the loss of carotenoid due to heat, the total content of the ascorbic acid compounds in the emulsion composition of the invention is preferably from 0.05 times to 50 times, more preferably from 1 time to 10 times, still more preferably from 1.5 times to 10 times, and still furthermore preferably from 2 times to 10 times the content of the carotenoid component including lycopene by mass. In a case in which the content of the ascorbic acid compound is 0.05 or more times the content of crystalline carotenoid including lycopene by mass, the effect of suppressing the decrease of the crystalline carotenoid including lycopene is sufficiently exhibited, and in a case in which the content of the ascorbic acid compound is 50 or less times, the formulation of crystalline carotenoid including lycopene in a sufficient amount cannot be impaired.

In the emulsion composition of the invention, the antioxidant may be used singly, or may be used in combination of two or more kinds.

Further, it is preferable to use a compound having a phenolic hydroxyl group together with an ascorbic acid compound, as an antioxidant, because the decomposition (for example, oxidative decomposition) due to the heating of carotenoid component including lycopene is surely suppressed, and the reduction of carotenoid component in the production process of emulsion composition can be suppressed.

[Preferred Embodiment of Oil-in-water Emulsion Composition]

Examples of the preferred embodiment of emulsion composition of the invention include an oil-in-water emulsion composition including dispersed particles that has an average particle diameter of 100 nm or less and contains a predetermined amount of lycopene, and at least one kind of specific additive. The emulsion composition of the invention in such an embodiment is excellent in the stability of fineness of dispersed particles and the transparency.

Further, the average particle diameter of dispersed particles in the emulsion composition means a particle diameter of the dispersed particles (oil droplets) in a composition.

The average particle diameter of dispersed particles in the invention is preferably 100 nm or less, and more preferably from 30 nm to 100 nm from the viewpoint of transparency and absorption of lycopene, and still more preferably from 35 nm to 85 nm, and most preferably from 40 nm to 70 nm from the viewpoint of transparency.

In view of the particle diameter range and the easiness of measurement, a dynamic light scattering method is preferable as the measurement method of average particle diameter of dispersed particles in the invention. Examples of the commercially available measurement device using dynamic light scattering include NANOTRAC UPA (Nikkiso Co., Ltd.), dynamic light-scattering method particle diameter distribution measuring apparatus LB-550 (Horiba, Ltd.), and concentrated system particle diameter analyzer FPAR-1000 (Otsuka Electronics Co., Ltd.). As the particle diameter in the invention, a value obtained by the measurement at 25°C using the particle diameter analyzer FPAR-1000 (Otsuka Electronics Co., Ltd.) is employed. Specifically, dilution with pure water is performed so that the oil phase concentration in the emulsion composition becomes 0.5% by mass, if necessary, and thus the particle diameter is determined as a median diameter (d = 50) using the particle diameter analyzer "FPAR-1000 (Otsuka Electronics Co., Ltd.)".

As described above, the emulsion composition of the invention is obtained by emulsification-mixing of an aqueous phase composition composed of an aqueous phase component and an oil phase composition constituted by an oil phase component.

From the viewpoint of functioning of the oil component, the content of oil phase composition in the emulsion composition is preferably from 0.001% by mass to 50% by mass, more preferably from 0.005% by mass to 30% by mass, and still more preferably from 0.01% by mass to 25% by mass.

Further, the average particle diameter of dispersion particles can be adjusted by a factor such as a stirring condition (shear force, temperature, or pressure) in the production method, or a ratio of oil phase and aqueous phase, other than the component of the composition.

In the emulsion composition of the invention, in addition to the components describe above, an emulsifier that can be used as the oil phase component may be contained. Examples of the emulsifier that can be used as the oil phase component include, for example, an emulsifier having an HLB of 7 or less among the emulsifiers described later.

In addition, the aqueous phase composition is constituted by an aqueous medium, particularly water, and contains preferably at least an emulsifier.

The emulsifier may be any of an anionic surfactant, a cationic surfactant, an ampholytic surfactant, and a nonionic surfactant.

Further, from the viewpoint of emulsifying power, the emulsifier has preferably an HLB of 10 or more, and more preferably 12 or more. In a case in which the HLB is extremely low, the emulsifying power may become insufficient. In addition, from the viewpoint of foam-suppressing effect, an emulsifier with HLB = 5 or more but less than 10 may be used in combination.

Herein, HLB indicates the hydrophilicity-hydrophobicity balance usually used in the field of surfactant, and can be calculated using a calculation equation usually used, for example, Kawakami equation. The Kawakami equation is described in the following.

$$HLB = 7 + 11.7 \log (Mw/Mo)$$

In the equation, Mw represents the molecular weight of hydrophilic group, and Mo represents the molecular weight of hydrophobic group.

Further, the HLB values described in a catalog or the like may be used.

As is understood from the above-described equation, an emulsifier having an arbitrary HLB value can be obtained by utilizing the additivity of HLB.

In the preparation of the emulsion composition of the invention, the content of the emulsifier at the time of adjusting the particle diameter of dispersed particles to preferably 100 nm or less by performing emulsification operation is preferably from 0.5% by mass to 30% by mass, more preferably from 1% by mass to 20% by mass, and still more preferably from 2% by mass to 15% by mass of the total mass of the emulsion composition. In the case of the range described above, the interfacial tension between an oil phase and a poor solvent phase is easily reduced, without becoming the excessive amount, and a problem of poor foaming of dispersion composition hardly occurs. Therefore, the content range is preferable.

Further, the total mass of the emulsifier can be used in the range of from 0.1 times to 10 times the total mass of the oil component containing a carotenoid component, and is preferably from 0.5 times to 8 times, and particularly preferably from 0.8 times to 5 times, in view of the refinement of emulsified particles and the suppression of foaming. In a case in which the emulsifier is used in the range described above, the dispersion stability of the composition can be favorable.

Among the emulsifiers, a nonionic surfactant is preferable because of the low irritation and the low impact to the environment. Examples of the nonionic surfactant include a sucrose fatty acid ester, a polyglycerin fatty acid ester, an organic acid monoglyceride, a propylene glycol fatty acid ester, a polyglycerin condensed ricinoleic acid ester, a sorbitan fatty acid ester, and a polyoxyethylene sorbitan fatty acid ester.

From the viewpoint of stability of the dispersed particles in the composition, in the sucrose fatty acid ester, the number of carbon atoms of the fatty acid constituting the sucrose fatty acid ester is preferably from 12 to 20, and more preferably from 14 to 16.

Preferred examples of the sucrose fatty acid ester include sucrose dioleate, sucrose distearate, sucrose dipalmitate, sucrose dimyristate, sucrose dilaurate, sucrose monooleate, sucrose monostearate, sucrose monopalmitate, sucrose monomyristate, and sucrose monolaurate. Among them, sucrose monooleate, sucrose monostearate, sucrose monopalmitate, sucrose monomyristate, and sucrose monolaurate are more preferable.

In the invention, these sucrose fatty acid esters can be used alone or in combination.

In the aqueous phase composition, a polyglycerin fatty acid ester may be contained in addition to the predetermined polyglycerin fatty acid ester described above.

Such a polyglycerin fatty acid ester is an ester of polyglycerin having an average degree of polymerization of 2 or more, preferably from 6 to 15, and more preferably from 8 to 10, and a fatty acid having from 8 to 18 carbon atoms, for example, caprylic acid, capric acid, lauric acid, myristic acid, palmitic acid, stearic acid, oleic acid, and linoleic acid.

Preferred examples of the polyglycerin fatty acid ester include hexaglycerin monooleate, hexaglycerin monostearate, hexaglycerin monopalmitate, hexaglycerin monomyristate, hexaglycerin monolaurate, decaglycerin monooleate, decaglycerin monostearate, decaglycerin monopalmitate, decaglycerin monomyristate, and decaglycerin monolaurate.

Among them, decaglycerin monooleate (HLB = 12), decaglycerin monostearate (HLB = 12), decaglycerin monopalmitate (HLB = 13), decaglycerin monomyristate (HLB = 14), decaglycerin monolaurate (HLB = 16), and the like are more preferable.

These polyglycerin fatty acid esters may be used alone or in combination.

In the sorbitan fatty acid ester in the invention, the number of carbon atoms of the fatty acid is preferably 8 or more, and more preferably 12 or more. Preferred examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, and sorbitan trioleate.

In the invention, these sorbitan fatty acid esters may be used alone or in combination.

In the polyoxyethylene sorbitan fatty acid ester, the number of carbon atoms of the fatty acid is preferably 8 or more, and more preferably 12 or more. Further, the length (the number of added moles) of ethylene oxide in polyoxyethylene is preferably from 2 to 100, and more preferably from 4 to 50.

Preferred examples of the polyoxyethylene sorbitan fatty acid ester include polyoxyethylene sorbitan monocaprylate, polyoxyethylene sorbitan monolaurate, polyoxyethylene sorbitan monostearate, polyoxyethylene sorbitan sesquistearate, polyoxyethylene sorbitan tristearate, polyoxyethylene sorbitan isostearate, polyoxyethylene sorbitan sesquiisostearate, polyoxyethylene sorbitan oleate, polyoxyethylene sorbitan sesquioleate, and polyoxyethylene sorbitan trioleate.

These polyoxyethylene sorbitan fatty acid esters may be used alone or in combination.

Furthermore, as an emulsifier in the invention, a phospholipid such as lecithin may also be contained.

The phospholipid that can be used in the invention contains a glycerin skeleton, and a fatty acid residue and a phosphate residue as essential components, to which a base, a polyhydric alcohol, and the like are bound, and is also referred to as lecithin. The phospholipid has a hydrophilic group and a hydrophobic group in the molecule, and thus conventionally has been used widely as an emulsifier in the fields of a food product, a pharmaceutical product, and a cosmetic.

A substance having a lecithin purity of 60% or more is industrially used as lecithin, and can also be used in the invention. From the viewpoint of the formation of an oil droplet having a fine particle diameter, and the stability of a functional oil component, the substance is generally referred to as high-purity lecithin, and has a lecithin purity of preferably 80% or more, and more preferably 90% or more.

Examples of the phospholipid may include conventionally-known various phospholipids that are obtained by extraction and separation from the living body of a plant, an animal or a microorganism.

Specific examples of such the phospholipid include, for example, various lecithins derived from a plant such as soybean, corn, peanut, rapeseed, or wheat; an animal such as an egg yolk, or a bovine; a microorganism such as Escherichia coli; or the like.

Examples of the lecithin include glycerinecithin such as phosphatidic acid, phosphatidylglycerin, phosphatidylinositol, phosphatidylethanolamine, phosphatidylmethylethanolamine, phosphatidylcholine, phosphatidylserine, bisphosphatidic acid, or diphosphatidylglycerin (cardiolipin); and sphingolecithin such as sphingomyelin, when described using a compound name.

Further, in the invention, hydrogenated lecithin, enzyme-degraded lecithin, enzyme-degraded hydrogenated lecithin, hydroxylecithin, and the like may be used in addition to the high purity lecithin described above. These lecithins that can be used in the invention may be used alone, or in the form of a mixture of the plural kinds.

[Other Addition Components]

In addition to each component described above, a component that is generally used in the fields of a food product, a cosmetic, and the like may also be appropriately mixed into the emulsion composition of the invention depending on the form of the composition. The addition component may be mixed as a component of the oil phase component mixture, the emulsion composition, or the aqueous phase composition depending on the properties of the addition component, or may be mixed as an addition component to the aqueous phase of the emulsion composition.

Examples of such other components may include glycerin; other polyhydric alcohols; a monosaccharide or a polysaccharide such as glucose, fructose, lactose, maltose, sucrose, pectin, kappa-carrageenan, locust bean gum, guar gum, hydroxypropyl guar gum, xanthan gum, karaya gum, tamarind seed polysaccharide, gum arabic, tragacanth gum, hyaluronic acid, sodium hyaluronate, sodium chondroitin sulfate, or dextrin; sugar alcohol such as sorbitol, mannitol, maltitol, lactose, maltotriitol, or xylitol; an inorganic salt such as sodium chloride, or sodium sulfate; a protein having a molecular weight of more than 5,000 such as casein, albumin, methylated collagen, hydrolyzed collagen, water-soluble collagen, or gelatin; a synthetic polymer such as a carboxyvinyl polymer, sodium polyacrylate, polyvinyl alcohol, polyethylene glycol, or an ethylene oxide-propylene oxide block copolymer; a water-soluble cellulose derivative such as hydroxyethyl cellulose methyl cellulose; a flavonoid (catechin, anthocyanin, flavone, isoflavone, fravane, flavanone, and rutin); a phenolic acid (chlorogenic acid, ellagic acid, gallic acid, and propyl gallate); a lignane; a curcumin; and a coumarin. Based on the function thereof, such a component may be contained as, for example, a functional component, an excipient, a viscosity modifier, and a radical scavenger.

In addition, other additives that are generally used for the application of, for example, various medicinal components, a pH adjuster, a pH buffer, an ultraviolet ray absorber, an antiseptic agent, perfume, or a coloring agent, may be used in combination.

The emulsion composition of the invention is an emulsion composition in which the decomposition of lycopene is suppressed, and the intended effect due to the lycopene is fully expected. Accordingly, the emulsion composition can be preferably applied to a food composition, in addition to a cosmetic composition, and an external preparation for skin such as a pharmaceutical composition.

Further, into a food product or a cosmetic that contains the emulsion composition of the invention, a component that can be added to a food product or a cosmetic may be appropriately added, if necessary.

The emulsion composition of the invention hardly has the decomposition of lycopene, therefore, in a case in which the emulsion composition is applied to a food product, a cosmetic, and the like, a favorable property such as favorable absorbability of lycopene can be realized.

As a cosmetic composition, the emulsion composition of the invention can be suitably used for a cosmetic composition, for example, lotion, beauty essence, milky lotion, massage mask, facial mask, a shampoo cosmetic, a fragrance cosmetic, a liquid body cleaning preparation, a UV care cosmetic, a deodorant cosmetic, and an oral care cosmetic, and the like.

Further, as the food product, the emulsion composition of the invention can be suitably used for a common food such as an energy drink, a nutritional supplement, a favorite beverage, or frozen dessert, and the like.

The emulsion composition of the invention can be produced according to a known method. Examples of the preferred production method of the emulsion composition according to the invention include the production method described in the following.

[Method of Producing Emulsion Composition]

The emulsion composition of the invention is preferably produced according to a production method including mixing an oil phase composition containing lycopene and an aqueous phase composition, and performing pressure emulsification.

In the production method of emulsion composition, the specific additive may be added to the aqueous phase composition before the oil phase composition is mixed, or may be added after the oil phase composition and the aqueous phase composition are mixed each other and then the pressure emulsification is performed.

Examples of the one of preferred embodiments for obtaining an oil phase composition containing lycopene include an embodiment including heating the oil phase component mixture containing lycopene; a (poly)glycerin fatty acid ester having from 1 to 3 glycerin units, from 1 to 6 fatty acid units, and at least one hydroxyl group from a glycerin unit; a fatty acid ester component having from 10 to 60 carbon atoms in total, which is at least one kind selected from the group consisting of a triester of glycerin and fatty acid, and an ester of alcohol having one hydroxyl group and fatty acid, and does not have a hydroxyl group in the molecule; and an antioxidant at a temperature of 90°C or higher.

Hereinafter, the production method of the emulsion composition will be described in detail by way of example of the production method of the emulsion composition using an oil phase composition of the embodiment, but the invention is not limited thereto.

According to the production method of the embodiment, lycopene is heated at a temperature of the melting temperature or more of carotenoid component, together with the predetermined (poly)glycerin fatty acid ester, the predetermined fatty acid ester component, and an antioxidant, and thus the carotenoid component is co-dissolved together with the predetermined (poly)glycerin fatty acid ester, and the predetermined fatty acid ester component. By using the oil phase composition that is subjected to heating emulsification together with an aqueous phase composition containing a water-soluble emulsifier as an oil phase composition containing lycopene that is obtained by the co-dissolution, an emulsion composition obtained by the emulsification becomes a composition in which the crystallization of the lycopene that is crystalline carotenoid is suppressed. The production method of the embodiment is to add the predetermined fatty acid ester component described above. As a result, as compared with a case in which the predetermined fatty acid ester component is not added, the dissolution temperature of lycopene can be decreased.

In the production method of the embodiment, an oil phase component mixture can be obtained by mixing firstly a carotenoid component containing at least one kind of crystalline carotenoid; a (poly)glycerin fatty acid ester having from 1 to 3 glycerin units, from 1 to 6 fatty acid units, and at least one hydroxyl group from a glycerin unit; a fatty acid ester component having from 10 to 60 carbon atoms in total, which is at least one kind selected from the group consisting of a triester of glycerin and fatty acid, and an ester of alcohol having one hydroxyl group and fatty acid, and does not have a hydroxyl group in the molecule; and an antioxidant (hereinafter, sometimes referred to as "oil phase component mixing process"). The oil phase component mixture may contain other oil phase components as necessary.

The specific examples of lycopene, a (poly)glycerin fatty acid ester, a fatty acid ester component, an antioxidant, and other oil components, which are contained in the oil phase component mixture and used in the oil phase component mixing process; the content; and the preferred range thereof, are the same as those in the description about each component contained in the emulsion composition of the invention.

After that, by heating the oil phase component mixture at a temperature of 90°C or higher, an oil phase composition can be obtained (hereinafter, sometimes referred to as "oil phase component heating process"). The heating temperature may be 90°C or higher, and can be from 90°C to 155°C. From the viewpoint of suppressing the thermal decomposition of carotenoid, the heating temperature is preferably from 110°C to 150°C, and more preferably from 120°C to 145°C.

The heating time in the oil phase component heating process may be the time during which lycopene in the oil phase component mixture is dissolved, and from the viewpoint of the efficient non-crystallization of crystals and the suppression of the decomposition of carotenoid due to excessive heat, is preferably from 1 minute to 60 minutes, and more preferably from 3 minutes to 45 minutes, but not limited thereto.

According to such a heating treatment, an oil phase composition can be obtained from the oil phase component mixture containing lycopene.

Further, in the heating treatment, it is preferable to adjust the temperature of entire oil phase component mixture to a uniform temperature, therefore, it is preferable to sufficiently stir the oil phase component mixture while heating, and it is desirable to maintain the temperature at a constant temperature by heating while stirring the mixture using a closed container.

According to the oil phase component heating process, the oil phase composition can be obtained.

Emulsification (emulsifying process) of the oil phase composition obtained according to the oil phase component heating process, and the aqueous phase composition (that may contain a specific additive) is included after the oil phase component heating process. As a result, an oil-in-water emulsion composition in which oil phase components containing lycopene is finely dispersed in water as oil droplets (dispersed particles) can be obtained. In the composition, lycopene is stability maintained.

The ratio (by mass) of the oil phase and the aqueous phase in the emulsification is not particularly limited, but is preferably from 0.1/99.9 to 50/50, more preferably from 0.5/99.5 to 30/70, and still more preferably from 1/99 to 20/80 by the ratio of oil phase/aqueous phase (mass %).

By setting the ratio of oil phase/aqueous phase to 0.1/99.9 or more, the active components are not decreased, therefore, it tends that a practical problem of oil-in-water emulsion composition does not occur, and thus which is preferable. Further, by setting the ratio of oil phase/aqueous phase to 50/50 or less, the concentration of the emulsifier is not decreased, therefore, it tends that the emulsification stability of oil-in-water emulsion composition is not deteriorated, and thus which is preferable.

The pressure emulsification may be performed by one-step operation of the emulsification, but is preferably performed by two or more-step operation of the emulsification in view of obtaining uniform and fine dispersed particles (emulsified particles).

Specifically, it is particularly preferable to use two or more kinds of emulsification devices in combination in a manner such that emulsification is performed by a high-pressure homogenizer or the like, in addition to the one-step operation of the emulsification in which emulsification is performed using an ordinary emulsification apparatus (for example, a stirrer, an impeller stirring, a homomixer, a continuous-flow type shearing apparatus, or the like) utilizing a shearing action. By using a high-pressure homogenizer, the emulsion can be aligned as droplets of more uniform and fine particles. Further, plural operations may be additionally performed in order to obtain the droplets having more uniform particle diameter.

As the emulsification means that can be used herein, any of generally known emulsification methods, such as a spontaneous emulsification method, a surface chemical emulsification method, an electric emulsification method, a capillary emulsification method, a mechanical emulsification method, or an ultrasonic emulsification method, can be used.

As a useful method of making the emulsified particles finer in the emulsion composition, a surface chemical emulsification method such as a PIT emulsification method, or a gel emulsification method has been known. This method has an advantage in that the consumption energy is small, and thus is suitable for the fine emulsification of a material that is easily deteriorated by heat.

Further, as an emulsification method that is used for general purposes, a method using a mechanical force, that is, a method of splitting oil droplets by applying a strong shearing force from the outside is applied. The most common machine of mechanical force is a high-speed and high-shearing stirring machine. As such a stirring machine, a stirring machine that is called a homomixer, a disper mixer, or an ultramixer is commercially available.

Further, as another mechanical emulsification apparatus that is useful for the micronizing, there is a high-pressure homogenizer, and various kinds of apparatuses are commercially available. The high-pressure homogenizer can provide greater shearing force as compared with that in a stirring method, therefore, even if the amount of the emulsifier is relatively small, the micronizing cab be performed.

The high-pressure homogenizer is roughly divided into a chamber type high-pressure homogenizer having a fixed throttle portion, and a homogeneous valve type high-pressure homogenizer in which the opening degree of throttle is controlled.

Examples of the chamber type high-pressure homogenizer include MICROFLUIDIZER (manufactured by Microfluidics Corporation), NANOMIZER (manufactured by Yoshida Kikai Co., Ltd.), and ULTIMIZER (manufactured by Sugino Machine Limited).

Examples of the homogeneous valve type high-pressure homogenizer include Gaulin-type homogenizer (manufactured by APV), Rannie-type homogenizer (manufactured by Rannie), HIGH-PRESSURE HOMOGENIZER (manufactured by Niro Soavi), HOMOGENIZER (manufactured by SANWA MACHINERY TRADING CO., LTD.), HIGH-PRESSURE HOMOGENIZER (manufactured by IZUMI FOOD MACHINERY CO., LTD.), and ULTRAHIGH-PRESSURE HOMOGENIZER (manufactured by IKA Corporation).

As a dispersing apparatus having a relatively favorable energy efficiency and an emulsification apparatus having a simple structure, there is an ultrasonic homogenizer. Examples of the high-power ultrasonic homogenizer that is capable of production include ultrasonic homogenizer US-600, ibid. US-1200T, ibid. RUS-1200T, and ibid. MUS-1200T (all manufactured by NIHONSEIKI KAISHA LTD.), and ultrasonic processor UIP 2000, ibid. UIP 4000, ibid. UIP 8000, and ibid. UIP 1600 (all manufactured by Heilscher). These high-power ultrasonic homogenizers are used at a frequency of 25 kHz or less, and preferably at a frequency of from 15 to 20 kHz.

Further, as other known emulsification means, a method using an apparatus such as a static mixer, a micro channel, a micro mixer, or a membrane emulsification apparatus, which does not include a stirring portion from the outside and requires only low energy, is also useful.

In the production method of the embodiment, the temperature condition at the time of emulsification and dispersion is not particularly limited but, from the viewpoint of stability of a functional oil component, is preferably from 10°C to 100°C, and the preferable range can be appropriately selected depending on the melting point and the like of the functional oil component to be handled.

Further, in the case of using a high-pressure homogenizer in the invention, the processing is preferably performed at a pressure of preferably 50 MPa or more, more preferably from 50 MPa to 280 MPa, and still more preferably from 100 MPa to 280 MPa.

Further, from the viewpoint of keeping a particle diameter of the emulsified particles, it is preferable that an emulsified liquid that is an emulsified and dispersed composition is cooled through some sort of cooler within 30 seconds, preferably within 3 seconds immediately after passing through a chamber.

The average particle diameter of dispersed particles in the emulsion composition obtained according to the production method described above is preferably 100 nm or less, and more preferably from 30 nm to 100 nm from the viewpoint of transparency and absorbability, and still more preferably from 35 nm to 85 nm, and most preferably from 40 nm to 70 nm from the viewpoint of transparency.

EXAMPLES

The invention will be described below with reference to examples, but the invention is not limited thereto. Note that the numerical numbers expressed by "part" or "%" in the following description is based on mass, unless otherwise specified.

[Examples 1 to 10, and Comparative Examples 1 to 6]

The lycopene-containing emulsions 1 to 10 that are the emulsion compositions of Examples 1 to 10, and the lycopene-containing emulsions C1 to C6 that are the emulsion compositions of Comparative Examples 1 to 6 were prepared as follows.

1. Preparation of Lycopene-containing Emulsion Composition

<Preparation of Lycopene-containing Emulsion A>

The following aqueous phase composition was heated and mixed while stirring in a constant temperature bath at 70°C, and was confirmed to be mixed well. The resultant aqueous phase composition was kept at 70°C. The following oil phase composition was heated and mixed for 5 minutes while stirring on a hot plate at 135°C, and was confirmed to be mixed well.

The aqueous phase composition was added into the oil phase composition, and mixed and stirred, and then the mixture was dispersed using an ultrasonic homogenizer. After that, the obtained crude dispersion was further subjected to high-pressure emulsification at 200 MPa using an ultra-high pressure emulsifier (ULTIMIZER, manufactured by Sugino Machine Limited).

As a result, lycopene-containing emulsion A was obtained.

-Aqueous Phase Composition-

| | |
|---|---|
| (1) Decaglyceryl oleate[1] | 10 parts |
| (2) Glycerin | 45 parts |
| (3) Purified water | 30 parts |

[1]: "Decaglyn 1-O" manufactured by Nikko Chemicals Co., Ltd.

-Oil Phase Composition-

| | |
|---|---|
| (1) Tomato oleoresin (the content of lycopene 6%)[2] | 8.4 parts |
| (2) Diglyceryl monostearate[2] | 0.3 part |
| (3) Tri(caprylic acid/capric acid)glycerin[4] | 5.7 parts |
| (4) Mixed tocopherol[5] | 0.6 part |

[2]: "Lyc-O-Mato 6%" manufactured by SUNBRIGHT CO., LTD.
[3]: "NIKKOL DGMS" (HLB = 5.0) manufactured by Nikko Chemicals Co., Ltd.
[4]: "COCONARD MT" (HLB = 1) manufactured by Kao Corporation
[5]: "Riken E Oil 800" manufactured by RIKEN VITAMIN CO., LTD.

<Preparation of Lycopene-containing Emulsions B to D>

Lycopene-containing emulsions B to D were prepared in the same manner as in the lycopene-containing emulsion A, except that the kind and content of each component, which are used in the oil phase composition and the aqueous phase composition in the preparation of lycopene-containing emulsion A, were changed as shown in Table 1.

A number indicating the mixing amount of each component in Table 1 represents "parts".

[Table 1]

| | | Lycopene-containing emulsion A | Lycopene-containing emulsion B | Lycopene-containing emulsion C | Lycopene-containing emulsion D |
|---|---|---|---|---|---|
| Oil phase composition | Tomato oleoresin (the content of lycopene: 6%) | 8.4 | 6.2 | 2.8 | 0.13 |
| | Diglyceryl monostearate | 0.3 | 0.3 | 0.3 | 0.3 |
| | Tri(caprylic acid/ capric acid)glycerin | 5.7 | 7.9 | 11.3 | 13.97 |
| | Mixed tocopherol | 0.6 | 0.6 | 0.6 | 0.6 |

(continued)

|  | | Lycopene-containing emulsion A | Lycopene-containing emulsion B | Lycopene-containing emulsion C | Lycopene-containing emulsion D |
|---|---|---|---|---|---|
| Aqueous phase composition | Decaglyceryl oleate | 10.0 | 10.0 | 10.0 | 10.0 |
| | Glycerin | 45.0 | 45.0 | 45.0 | 45.0 |
| | Purified water | 30.0 | 30.0 | 30.0 | 30.0 |
| Particle diameter immediately after the preparation | | 59nm | 56nm | 55nm | 57nm |
| Lycopene-containing emulsion A: the lycopene content in dispersed particles is 3.4%<br>Lycopene-containing emulsion B: the lycopene content in dispersed particles is 2.5%<br>Lycopene-containing emulsion C: the lycopene content in dispersed particles is 1.1%<br>Lycopene-containing emulsion D: the lycopene content in dispersed particles is 0.05% | | | | | |

Each particle diameter immediately after the preparation of the lycopene-containing emulsions A to D obtained in the above was measured. The measurement results are shown in Table 1.

Each of the lycopene-containing emulsions A to D was filled into a 5 mL glass vial, and thus a sample was produced. Purified water was added into each sample to prepare a 0.5% dilution. The average particle diameter (median particle diameter) of the particles in each dilution was measured on a volume basis with a dynamic light scattering meter (FPAR-1000 manufactured by Otsuka Electronics Co., Ltd.).

<Preparation of Lycopene-containing Emulsions 1 to 10 and C1 to C6>

(1) The lycopene-containing emulsion A, B, C, or D obtained in the above , (2) the specific additive shown in the following Table 2, and (3) purified water were used to be mixed each other while stirring at 25°C so as to obtain the composition described in the following Table 2, and thus the lycopene-containing emulsions 1 to 10 for Examples 1 to 10, and the lycopene-containing emulsions C1 to C6 for Comparative Examples 1 to 6 were prepared.

2. Evaluation

For the lycopene-containing emulsions 1 to 10 and C1 to C6 obtained in the above, the following evaluations (1) to (4) were conducted, as the evaluation of the suppression of the decomposition of lycopene, and the evaluation of the storage stability of the composition. The evaluation results are shown in Table 2.

(1) Lycopene Residual Rate

The lycopene-containing emulsions 1 to 10 and C1 to C6 obtained in the above were measured by a liquid chromatography method immediately after the preparation, and after two-week storage at 40°C.

The intensity ratio immediately after the preparation and after the storage was evaluated as the residual rate of lycopene in each lycopene-containing emulsion composition with the value immediately after the preparation being designated as 100%.

(2) Color Change of Emulsion Composition after Storage

The lycopene-containing emulsions 1 to 10 and C1 to C6 obtained in the above were stored at 40°C for 2 weeks, and then the color change was visually confirmed and evaluated according to the following evaluation criteria.

<Evaluation Criteria>

A: No color change is observed.

B: Change is small, but color change is observed slightly.
C: Color change is clearly observed.

(3) Particle Diameter of Dispersed Particles

Each of the lycopene-containing emulsions 1 to 10 and C1 to C6 obtained in the above was divided into two portions, each portion was filled into a 5 mL glass vial, and thus a sample was produced. One of the two samples was stored at 40°C for 2 weeks. For each sample immediately after the preparation, or after the two-week storage, the average particle diameter (median particle diameter) of the particles in each sample was measured on a volume basis with a dynamic light scattering meter (FPAR-1000 manufactured by Otsuka Electronics Co., Ltd.).

(4) Transparency

Each 1% dilution of the lycopene-containing emulsions 1 to 10 and C1 to C6 obtained in the above immediately after the preparation, or after two-week storage at 40°C, was placed in a vessel with a height of 5 cm or more so that the distance from the bottom of the vessel to the liquid surface was 4cm, and was visually observed from vertically above the liquid surface toward the bottom of the vessel through the sample solution. In this manner, the transparency was evaluated according to the criteria shown in the following by using observation of whether the bottom of the vessel was visually observed or not as an evaluation of the transparency.

<Evaluation Criteria>

A:    The bottom is clearly observed.
B:    The bottom is vaguely observed.
C:    The bottom is not observed.

[Table 2]

| | | Example 1 | Example 2 | Example 3 | Example 4 | Example 5 | Example 6 | Example 7 | Example 8 | Example 9 | Example 10 | Comp Example 1 | Comp. Example 2 Comp. Example 3 | Comp. Example 4 | Comp. Example 5 | Comp. Example 6 |
|---|---|---|---|---|---|---|---|---|---|---|---|---|---|---|---|---|
| (1) | Lycopene-containing emulsion A (Lycopene content in dispersed particles: 3.4%) | | | | | | | | | | | 0.50 | | | 0.50 | |
| | Lycopene-containing emulsion B (lycopene content in dispersed particles: 2.5%) | 0.50 | | | | | | | | | | | 0.50 | | | |
| | Lycopene-containing emulsion C (Lycopene content in dispersed particles: 1.1%) | | 0.50 | | 0.50 | 0.50 | 0.50 | 0.50 | 0.50 | 0.50 | 0.50 | | | 0.50 | | 0.50 |
| | Lycopene-containing emulsion D (Lycopene content in dispersed particles: 0.05%) | | | 0.50 | | | | | | | | | | | 0.50 | |
| (2) | Methyl parahydroxybenzoate | | | | | | | | | | | | | | | 0.20 |
| | GLYCACIL2000 (Iodopropynyl butvlcarbamate: 6%) | 0.10 | 0.10 | 0.10 | | | | | | | | | | | | |
| | Ethylhexyl glycerin | | | | 0.20 | | | | | | | | | | | |
| | 1,3-Butanediol | | | | | 15.00 | | | | | | | | | | |
| | 1,2-Pentanediol | | | | | | 10.00 | | | | | | | | 10.00 | |

EP 2 829 263 B1

18

(continued)

| | | Example 1 | Example 2 | Example 3 | Example 4 | Example 5 | Example 6 | Example 7 | Example 8 | Example 9 | Example 10 | Comp Example 1 | Comp. Example 2 | Comp. Example 3 | Comp. Example 4 | Comp. Example 5 | Comp. Example 6 |
|---|---|---|---|---|---|---|---|---|---|---|---|---|---|---|---|---|---|
| | 1,6-Hexanediol | | | | | | | 7.00 | | | | | | | | | |
| | 1,2-Octanediol | | | | | | | | 1.00 | | | | | | | | |
| | 1,2-Decanediol | | | | | | | | | 0.50 | | | | | | | |
| | Phenoxyethanol | | | | | | | | | | 0.70 | | | | | | |
| (3) | Water | 99.40 | 99.40 | 99.40 | 99.30 | 84.50 | 89.50 | 92.50 | 98.50 | 99.00 | 98.80 | 99.50 | 99.50 | 99.50 | 99.50 | 89.50 | 99.30 |
| Evaluation results | Lycopene residual rate after the storage at 40°C for 2 weeks | 88% | 91% | 88% | 86% | 85% | 85% | 88% | 89% | 89% | 90% | 65% | 67% | 70% | 69% | 71% | 55% |
| | Color change after the storage at 40°C for 2 weeks | A | A | A | B | B | B | A | A | A | A | C | C | C | C | C | C |
| | Particle diameter immediately after the preparation | 56nm | 55nm | 57nm | 55nm | 55nm | 55nm | 55nm | 55nm | 55nm | 55nm | 59nm | 56nm | 55nm | 57nm | 59nm | 55nm |
| | Particle diameter after the storage at 40°C for 2 weeks | 56nm | 55nm | 57nm | 57nm | 65nm | 61nm | 60nm | 55nm | 55nm | 59nm | 105nm | 110nm | 103nm | 115nm | 62nm | 57nm |
| | Transparency after the storage at 40°C for 2 weeks | A | A | A | A | B | A | A | A | A | A | C | C | C | C | B | A |

EP 2 829 263 B1

As shown in Table 2, it is understood that all of the lycopene-containing emulsions 1 to 10, which are emulsion compositions of the Examples, are excellent in storage stability.

In particular, from comparison of Comparative Examples 1 to 5 with Examples 1 to 3, it is understood that the storage stability is significantly increased by the selective combination of the specific additive and the dispersed particles containing lycopene in a predetermined amount in the invention.

Further, the specific additive is a compound having an antiseptic effect, and it has in common with methyl parahydroxybenzoate, which is a widely used antiseptic agent, used in Comparative Example 6, the fact that it has an antiseptic effect. However, as is understood from comparison of Comparative Example 6 with Examples 2, 4 to 10, the lycopene-containing emulsion in Comparative Example 6 is, unlike the lycopene-containing emulsions in the Examples, remarkably inferior in the lycopene residual rate. From this fact, it is also understood that the selective combination of the specific additive and the dispersed particles containing lycopene in a predetermined amount in the invention significantly contributes to improvement of the stability of lycopene.

## Claims

1. An oil-in-water emulsion composition, comprising:

   dispersed particles containing from 0.05% by mass to 2.5% by mass of lycopene relative to the total mass of the oil-phase; and
   a carbamic acid ester compound.

2. The oil-in-water emulsion composition according to Claim 1, wherein the dispersed particles have an average particle diameter of 100 nm or less.

3. The oil-in-water emulsion composition according to Claim 1 or 2, wherein the carbamic acid ester compound comprises iodopropynyl butylcarbamate.

4. An external preparation for skin, comprising the oil-in-water emulsion composition according to any one of Claims 1 to 3.

## Patentansprüche

1. Öl-in-Wasser-Emulsionszusammensetzung, umfassend:

   dispergierte Teilchen, die 0,05 Massen-% bis 2,5 Massen-% Lycopin enthalten, bezogen auf die Gesamtmasse der Ölphase; und
   eine Carbamidsäureesterverbindung.

2. Öl-in-Wasser-Emulsionszusammensetzung gemäß Anspruch 1, wobei die dispergierten Teilchen einen mittleren Teilchendurchmesser von 100 nm oder weniger aufweisen.

3. Öl-in-Wasser-Emulsionszusammensetzung gemäß Anspruch 1 oder 2, wobei die Carbamidsäureesterverbindung Iodpropinylbutylcarbamat umfasst.

4. Hautpräparat für die äußerliche Anwendung, umfassend die Öl-in-Wasser-Emulsionszusammensetzung gemäß einem der Ansprüche 1 bis 3.

## Revendications

1. Composition d'émulsion huile-dans-l'eau comprenant :

   des particules dispersées contenant de 0,05% en masse à 2,5% en masse de lycopène par rapport à la masse totale de la phase huileuse ; et
   un composé ester d'acide carbamique.

2. La composition d'émulsion huile-dans-l'eau selon la revendication 1, dans laquelle les particules dispersées ont un diamètre de particule moyen de 100 nm ou moins.

3. La composition d'émulsion huile-dans-l'eau selon la revendication 1 ou 2, dans laquelle le composé ester d'acide carbamique comprend du butylcarbamate d'iodopropynyle.

4. Préparation externe pour la peau, comprenant la composition d'émulsion huile-dans-l'eau selon l'une quelconque des revendications 1 à 3.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008120712 A

- JP 2011241177 A

**Non-patent literature cited in the description**

- **KAJIMOTO.** Kosankazai no Riron to Jissai (Theory and Practice of Antioxidants). San Shobo, 1984

- **SAWATARI ; NISHINO ; TABATA.** Sanka Boshizai Handobukku (Handbook of Antioxidants). Taiseisha, 1976